# EUROPEAN PATENT APPLICATION

(11) **EP 2 380 867 A1**
(43) Date of publication of application: **26.10.2011**
(21) Application number: 10160198.7
(22) Date of filing: 16.04.2010
(51) Int. Cl.: C07C 65/38, C08G 73/10, C09J 179/08, C07D 307/89

(54) **Novel cross-linker**

(71) Applicant: Nexam Chemical AB, 221 84 Lund (SE)
(72) Inventor: Rosenberg, Jan-Erik, 311 41, Falkenberg (SE); Röme, Daniel, 211 50, Malmö (SE); Persson, David, 217 51, Malmö (SE); Lager, Erik, 227 62, Lund (SE)
(74) Representative: Ström & Gulliksson AB

(57) **Abstract**

Disclosed are novel cross-linkable end-cappers for oligo- and polyimides. End-capped oligo- and polyimides comprising such an end-capper may be cured at a lower temperature compared to oligo- and polyimides end-capped with PEPA.

## Description

### Field of the invention

The present invention refers to novel cross-linkable end-cappers for oligo- and polyimides, which end-cappers comprise carbon-carbon triple bonds. Further, the present invention refers to an end-capped oligo- or polyimide. It also relates to an article comprising the oligo- or polyimide, wherein the oligo- or polyimide optionally has been cross-linked by heating it.

### Background

Polymers has for long been used as replacement materials for other materials, such as metals. They have the advantage of being light-weight materiel, which are relative easy to shape. However, polymers do typically have lower mechanical strength compared to metals. Further, they are less heat resistant.

The need for resistant polymers led to the development of aromatic polyimides. Polyimides are polymers comprising imide bonds. Aromatic polyimides are typically synthesized by condensation of aromatic carboxylic acid dianhydride monomers, such as pyromellitic dianhydride, 4,4'-oxydiphthalic anhydride, 2,2-bis-[4-(3,4-dicarboxyphenoxy)phenyl]-propane dianhydride, 3,3',4,4'-benzophenonetetracarboxylic acid dianhydride or 3,3',4,4'-tetracarboxybiphenyl dianhydride, with aromatic diamine monomers, such as 4,4'-oxydianiline, 1,4-diaminobenzene, 1,3-diaminobenzene, 1,3-bis-(4-aminophenoxy)benzene, 1,3-bis-(3-aminophenoxy)benzene, methylenedianiline or 3,4'-oxydianiline.

Polyimides obtained via condensation of pyromellitic dianhydride and 4,4'-oxydianiline are among others sold under the trademarks Vespel® and Meldin®. They are materials which are lightweight and flexible, and which have good resistant to heat and chemicals.

Further, thermoset polyimides have inherent good properties, such as wear and friction properties, good electrical properties, radiation resistance, good cryogenic temperature stability and good flame retardant properties. Therefore, they are used in the electronics industry for flexible cables, as an insulating film on magnet wire and for medical tubing. Polyimide materials are also used in high or low temperature exposed applications as structural parts were the good temperature properties is a prerequisite for the function.

The need to improve the processability, while keeping the mechanical properties, of polyimides for use in airplanes and aerospace applications led to the introduction of cross-linking technologies. As the polymer chains are cross-linked, they may be shorter whilst the mechanical properties are maintained or even improved. Shorter polymer chains have the advantage of being easier to process, as the viscosity of the polymer melt is lower.

Examples of such cross-linking technologies include the bismaleimides and the nadimide-based PMR resins, which undergo cure at temperatures near 250°C. However, such thermoset polyimides will not withstand oxidative degradation on long-term exposure at temperatures above 200°C, as the crosslinking moieties have inferior thermal stability, compared to the oligoimide units.

In attempts to improve the thermal stability, thermoset polyimides containing phenylethynyl-substituted aromatic species as the reactive moieties have been developed.

US 5,567,800 discloses phenylethynyl terminated imide oligomers (PETIs). Such oligomers may be prepared by first preparing amino terminated amic acid oligomers from dianhydride(s) and an excess of diamine(s) and subsequently end-cap the resulting amino terminated amic acid oligomers with phenylethynyl phtalic anhydride (PEPA). The amic acid oligomers are subsequently dehydrated to the corresponding imide oligomers.

Upon heating the triple bonds will react and cross-link the end-capped polyimid, thereby further improving its heat resistance and mechanical strength. As disclosed by US 5,567,800 heating to at least 350°C is necessary to cure the PETI.

However, for some applications the high curing temperature may be considered a problem. For instance, the properties (such as the coefficient of thermal expansion) of flexible polyimide films, having a melting temperature below 350°C, may be improved via cross-linking. However, the high temperature (above 350 °C) needed to initiate cross-linking will make the processing impossible.

If the curing temperature may be lowered, a film may be formed from a solution. During the drying step, curing may then be initiated by heating the film without melting it.

As an alternative to PEPA, also ethynyl phtalic anhydride (EPA) has been used as cross-linker in polyimides (Hergenrother, P. M., "Acetylene-terminated Imide Oligomers and Polymers Therefrom", Polymer Preprints, Am. Chem. Soc., Vol. 21 (1), p. 81-83, 1980). Although polyimides comprising EPA may be cross-linked at a somewhat lower temperature, i.e. at about 250°C, it suffers from other drawbacks. The exchange of the phenyl ethynyl group to an ethynyl group implies that other reaction pathways than the desired curing mechanism, such as chain extension, are favored. As a consequence, EPA has not found any wide use as a replacement to PEPA as a low temperature curing end-capper. Further, the manufacture of EPA requires protective group chemistry hampering its commercial potential.

US 6,344,523 addresses the disadvantageous of the high curing temperature discussed above and discloses that use of sulfur or organic sulfur derivatives as curing promoters may lower the curing temperature of PETI. However, the introduction of such promotors suffers from other disadvantages. In particular the curing results in chain extension rather than cross-linking as two ethynyl groups react along with one sulfur radical ultimately forming a thiophene structure.

Thus, there is need within the art for an alternative cross-linking monomer, overcoming the above-mentioned deficiencies, to replace PEPA and EPA in poly- and oligoimides.

### Summary

Consequently, the present invention seeks to mitigate, alleviate, eliminate or circumvent one or more of the above-identified deficiencies in the art and disadvantages singly or in any combination by providing a compound according to formula (I) or (II) 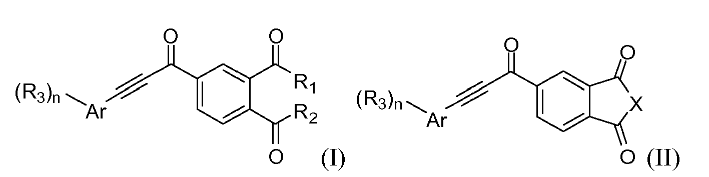 wherein
"Ar" is an aryl or a heteroaryl;
R1 and R2 are independently selected from the group consisting of OH, halo, OC1-C8 alkyl, NH2, NHC1-8 alkyl, N(C1-8 alkyl)₂, wherein said alkyl may be the same or different, OC(O)C1-8 alkyl, OC0-1 alkylenephenyl, and NHC0-1 alkylenephenyl;
R3 is, independently if "n" is 2 or more, selected from the group consisting of C1-4 alkyl, OC1-4 alkyl, halogen, cyano, nitro, C1-4 fluoroalkyl;
the substituent(s) R3 may be connected to any substitutable atom(s) of "Ar";
"n" is an integer from 0 (zero) to 5; and "X" is selected from the group consisting of O (oxygen), NH, N-phenyl, N-benzyl, and N-C1-8 alkyl.

Another aspect of the invention relates to an oligo- or polyimide comprising a residue according to formula (III), 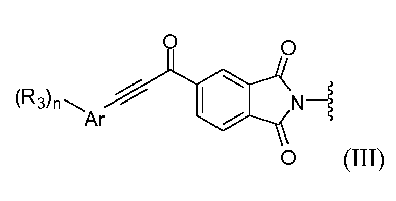 wherein
the waved line indicates the point of attachment to the oligo- or polyimide;
"Ar" is an aryl or a heteroaryl;
R3 is, independently if "n" is 2 or more, selected from the group consisting of C1-4 alkyl, OC1-4 alkyl, halogen, cyano, nitro, C1-4 fluoroalkyl;
the substituent(s) R3 may be connected to any substitutable atom(s) of "Ar"; and
"n" is an integer from 0 (zero) to 5. Typically, such an oligo- or polyimide comprises at least one residue of a monomer selected from the group consisting of pyromellitic dianhydride, 4,4'-oxydiphthalic anhydride, 2,2-bis-[4-(3,4-dicarboxyphenoxy)phenyl]-propane dianhydride, 3,3',4,4'-benzophenonetetracarboxylic acid dianhydride and 3,3',4,4'-tetracarboxybiphenyl dianhydride and at least one residue of a monomer selected from the group consisting of 4,4'-oxydianiline, 1,4-diaminobenzene, 1,3-diaminobenzene, 1,3-bis-(4-aminophenoxy)benzene, 1,3-bis-(3-aminophenoxy)benzene, methylenedianiline, and 3,4'-oxydianiline. The oligo- or polyimide may further have a number average molecular weight of about 1,000 to 20,000, such as from about 2,500 to 10,000.

Another aspect of the invention relates to composition comprising such an oligo- or polyimide. Such a composition may comprise an additional polymer, and/or at least one filler, reinforcement, pigment, and/or plasticizer. Typically, the composition comprises at least 10 wt% of the oligo- or polyimide.

Another aspect of the invention relates to method of producing a compound according to formula (II) as disclosed above. Such a method comprises the steps of:
- reacting trimellitic anhydride chloride with a compound according to formula (IV) 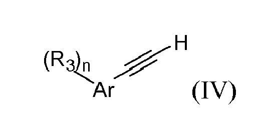
wherein
"Ar" is an aryl or a heteroaryl;
R3 is, independently if "n" is 2 or more, selected from the group consisting of C1-4 alkyl, OC1-4 alkyl, halogen, cyano, nitro, C1-4 fluoroalkyl;
the substituent(s) R3 may be connected to any substitutable atom(s) of "Ar"; and
"n" is an integer from 0 (zero) to 5.

Further advantageous features of the invention are defined in the dependent claims. In addition, advantageous features of the invention are elaborated in embodiments disclosed herein.

### Detailed summary of preferred embodiments

### Definitions:

In the context of the present application and invention, the following definitions apply:
As used herein, "halo" or "halogen" refers to fluoro, chloro, bromo, and iodo.
As used herein, "alkyl" used alone or as a suffix or prefix, is intended to include both branched and straight chain saturated aliphatic hydrocarbon groups having from 1 to 12 carbon atoms or if a specified number of carbon atoms is provided then that specific number is intended. For example "C1-6 alkyl" denotes alkyl having 1, 2, 3, 4, 5 or 6 carbon atoms. When the specific number denoting the alkyl-group is the integer 0 (zero), a hydrogen-atom is intended as the substituent at the position of the alkyl-group. For example, "N(C0 alkyl)₂" is equivalent to "NH2" (amino).
As used herein, "alkylenyl" or "alkylene" used alone or as a suffix or prefix, is intended to include straight chain saturated aliphatic hydrocarbon groups having from 1 to 12 carbon atoms or if a specified number of carbon atoms is provided then that specific number is intended. For example "C1-6 alkylenyl " "C1-6 alkylene "denotes alkylenyl or alkylene having 1, 2, 3, 4, 5 or 6 carbon atoms. When the specific number denoting the alkylenyl or alkylene-group is the integer 0 (zero), a bond is intended to link the groups onto which the alkylenyl or alkylene-group is substituted. For example, "NH(C0 alkylene)NH₂" is equivalent to "NHNH₂" (hydrazino). As used herein, the groups linked by an alkylene or alkylenyl-group are intended to be attached to the first and to the last carbon of the alkylene or alkylenyl-group. In the case of methylene, the first and the last carbon is the same. For example, "H₂N(C2 alkylene)NH₂", "H₂N(C3 alkylene)NH₂", "N(C4 alkylene)", "N(C5 alkylene)" and "N(C2 alkylene)₂NH" is equivalent to 1,2-diamino ethane, 1,3-diamino propane, pyrrolidinyl, piperidinyl and piperazinyl, respectively.

Examples of alkyl include, but are not limited to, methyl, ethyl, n-propyl, i-propyl, n-butyl, i-butyl, sec-butyl, t-butyl, pentyl, and hexyl.

Examples of alkylene or alkylenyl include, but are not limited to, methylene, ethylene, propylene, and butylene.

As used herein, "fluoroalkyl" and "fluoroalkylene", used alone or as a suffix or prefix, refers to groups in which one, two, or three of the hydrogen(s) attached to any of the carbons of the corresponding alkyl- and alkylene-groups are replaced by fluoro.

Examples of fluoroalkyl include, but are not limited to, trifluoromethyl, difluoromethyl, fluoromethyl, 2,2,2-trifluoroethyl, 2-fluoroethyl and 3-fluoropropyl.

Examples of fluoroalkylene include, but are not limited to, difluoromethylene, fluoromethylene, 2,2-difluorobutylene and 2,2,3-trifluorobutylene.

As used herein, the term "aryl" refers to a ring structure, comprising at least one aromatic ring, made up of from 5 to 14 carbon atoms. Ring structures containing 5, 6, 7 and 8 carbon atoms would be single-ring aromatic groups, for example phenyl. Ring structures containing 8, 9, 10, 11, 12, 13, or 14 carbon atoms would be polycyclic, for example naphthyl. The aromatic ring may be substituted at one or more ring positions. The term "aryl" also includes polycyclic ring systems having two or more cyclic rings in which two or more carbons are common to two adjoining rings (the rings are "fused rings") wherein at least one of the rings is aromatic, for example, the other cyclic rings may be cycloalkyls, cycloalkenyls, cycloalkynyls, and/or aryls.

The terms ortho, meta and para apply to 1,2-, 1,3- and 1,4-disubstituted benzenes, respectively. For example, the names 1,2-dimethylbenzene and orthodimethylbenzene are synonymous.

As used herein, "heteroaryl" refers to an aromatic heterocycle, having at least one ring with aromatic character, (e.g. 6 delocalized electrons) or at least two conjugated rings with aromatic character, (e.g. 4n + 2 delocalized electrons where "n" is an integer), and comprising up to about 14 carbon atoms, and having at least one heteroatom ring member such as sulfur, oxygen, or nitrogen. Heteroaryl groups include monocyclic and bicyclic (e.g., having 2 fused rings) systems.

As used herein, the term "substitutable" refers to an atom to which a hydrogen may be covalently attached, and to which another substituent may be present instead of the hydrogen. A non-limiting example of substitutable atoms include the carbon-atoms of pyridine. The nitrogen-atom of pyridine is not substitutable according to this definition.

### Embodiments

It has unexpectedly been revealed that compounds according to formula (I) or (II), 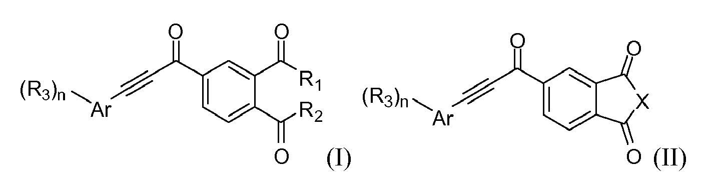 wherein
"Ar" is an aryl, such as phenyl or naphtyl, or a heteroaryl, such as thiophenyl or furanyl;
R1 and R2 are independently selected from the group consisting of OH, halo, such as chloro, OC1-C8 alkyl, such as OC1-C4 alkyl, NH2, NHC1-8 alkyl, N(C1-8 alkyl)₂, wherein said alkyl may be the same or different, OC(O)C1-8 alkyl, OC0-1 alkylenephenyl, and NHC0-1 alkylenephenyl;
R3 is, independently if "n" is 2 or more, selected from the group consisting of C1-4 alkyl, such as methyl, OC1-4 alkyl, such as methoxy, halogen, cyano, nitro, C1-4 fluoroalkyl, such as trifluoromethyl;
the substituent(s) R3 may be connected to any substitutable atom(s) of "Ar";
"n" is an integer from 0 (zero) to 5;
"Ar" is unsubstituted if "n" is 0 (zero); and
"X" is selected from the group consisting of O (oxygen), NH, N-phenyl, N-benzyl, and N-C1-8 alkyl;
may be used as end-cappers for oligo- and polyimides to obtain end-capped oligo- and polyimides that may be cross-linked at significantly lower temperatures compared to PETI.

Thus, an embodiment relates to a compound according to formula (I) or (II) as disclosed herein.

Typically, oligo- and polyimides comprising residues of compounds according to formula (I) or (II) may be cross-linked (cured) well below 300°C, such as at about 250°C. Thus, such oligo- and polyimides may be cross-linked at temperatures wherein also oligo- and polyimides comprising EPA are cross-linked.

However, in contrast to EPA, compounds according to formula (I) or (II) has no hydrogen atom directly attached to the carbon-carbon triple bond (acetylenic hydrogen). As a consequence, compared to EPA cross-linking is more favored for compounds according to formula (I) or (II) as non-oxidative head-to-head coupling (Straus coupling) is not possible for such compounds. In addition, the synthesis of compounds according to formula (I) or (II) are not dependent on protective group chemistry decreasing the complexity of their manufacture compared to EPA.

The preparation of compounds according to formula (I) or (II) are generally based on readily available raw materials, such as trimelletic anhydride and ethynylaryls, such as ethynylbensen.

Typically, trimelletic anhydride is activated by reaction with thionyl chloride to form an acid chloride, which is coupled to an ethynylaryl in a palladium catalyzed reaction.

It is to be noted that R1 and R2 of formula (I) and "X" of formula (II) also may represent more intricate groups. Thus, an embodiment relates to derivatives of compounds according to formula (I) or (II).

According to an embodiment "Ar" is an aryl, such as phenyl or napthyl. Preferably, "Ar" is phenyl. "Ar" may also be a functional equivalent to phenyl, such as thiophenyl.

If "Ar" is phenyl, then compounds according to formula (I) or (II) may be represented by formula (Ia) or (IIa), respectively, 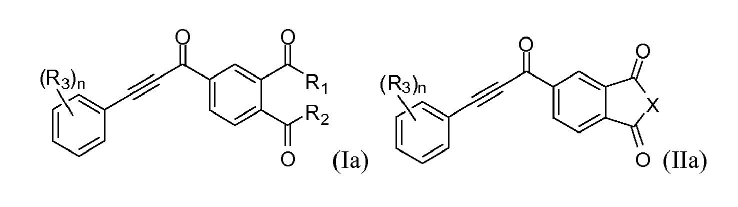 wherein R1, R2, R3 and "X" have the meaning indicted above for formula (I) and (II). In formula (Ia) and (IIa), R3 may connected to any of the substitutable carbon atoms in the phenyl group.

The integer "n" is preferably 0 (zero) or 1, such as being 0 (zero). If "n" is 1 or more, R3 is preferably, independently if "n" is 2 or more, selected from the group consisting of methyl, methoxy, nitro, and trifluoromethyl. As "n" may be 0 (zero), "Ar" may be un-substituted.

According to one embodiment "n" is 0 (zero) and "Ar" is phenyl.

Although the compound used in the manufacture of end-capped oligo- and polyimided may be a compound according to formula (I), the compound initially synthesized is typically a compound according to formula (II). In a compound according to formula (II), "X" is preferably O (oxygen), NH or NMe, such as being O (oxygen). As readily understood by the skilled artesian, compounds according to formula (II) may readily be converted into compounds according to formula (I), such as by reaction with mono-hydric alcohols.

According to one embodiment, compounds to be used as end-cappers are synthesized as compounds according to formula (II). These compounds may then be converted into compounds according to formula (I) prior to being used as end-cappers. In such compounds, R1 and R2 are preferably selected from OH, chloro, and OC1-C8 alkyl, such as methyl or ethyl. Preferably, R1 and R2 represent the same type of substituent.

In an embodiment wherein "Ar" is phenyl and "n" is one, the substituent may be in para-position ("Ar" thus representing 1,4-substituted benzene). Having a substituent in 2-position (orto), may increase the curing temperature and this may, dependent on the circumstances, be advantageous or disadvantageous.

In a preferred embodiment, the compound according to formula (I) or (II) is 5-(3-phenylprop-2-ynoyl)isobenzofuran-1,3-dione, also denoted PETA (PhenylEthynylTrimelleticAnhydride). PETA has the structure indicated below.

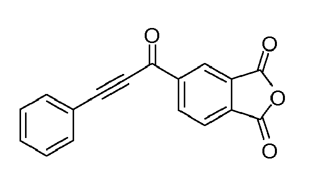

As compounds according to formula (I) or (II) are suitable as cross-linkable end-cappers for oligo- or polyimides, which may be cross-linked at low temperatures, such as below 300°C, another embodiment relates to an oligo- or polyimide comprising a residue according to formula (III), 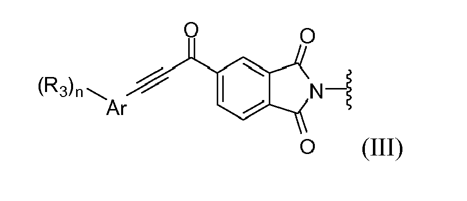 wherein the waved line indicates the point of attachment to the oligo- or polyimide, and "Ar", "n", and R3 have the same meaning as in formula (I) and (II).

The oligo- or polyimide to be end-capped may for example be a an amino terminated oligo- or polyimide, such as an amino terminated oligo- or polyimide obtainable by polymerization of an aromatic dianhydride with an slight excess of an aromatic diamine. The aromatic dianhydride may for example be pyromellitic dianhydride, 4,4'-oxydiphthalic anhydride, 2,2-bis-[4-(3,4-dicarboxyphenoxy)phenyl]-propane dianhydride, 3,3',4,4'-benzophenonetetracarboxylic acid dianhydride and 3,3',4,4'-tetracarboxybiphenyl dianhydride. The aromatic diamine may for example be 4,4'-oxydianiline, 1,4-diaminobenzene, 1,3-diaminobenzene, 1,3-bis-(4-aminophenoxy)benzene, 1,3-bis-(3-aminophenoxy)benzene, methylenedianiline, or 3,4'-oxydianiline.. Typically, the aromatic dianhydride is pyromellitic dianhydride and the aromatic diamine is 4,4'-oxydianiline or 1,3-diaminobenzene.

An oligo- or polyimide comprising a residue according to formula (III), may, according to one embodiment, have a number average molecular weight of about 1,000 to 20,000, such as from about 2,500 to 10,000. The number average molecular weight may be determined with gel permeation chromatography (GPC) or size exclusion chromatography (SEC).

Another embodiment relates to composition comprising an oligo- or polyimide comprising a residue according to formula (III). The composition may further comprise at least one additional polymer, such as at least one additional oligo- or polyimide, and/or at least one filler, reinforcement, pigment, plasticizer and/or any other additive known in the art. The oligo- or polyimide comprising a residue according to formula (III) is preferably present in an amount corresponding to at least 10 wt%, such as at least 25, 40, 60, or 80 wt% of the composition.

Another embodiment relates to an article comprising an oligo- or polyimide comprising a residue according to formula (III). Optionally, the oligo- or polyimide in the article has been cross-linked by heating it. Typically examples of articles include flexible films for electronics, wire isolation, wire coatings, wire enamels, ink, and load-bearing structural components.

As well known within the art, the preparation of oligo- and polyimides are preferably performed in, but not limited to, aprotic solvents, such as dimethylacetamide, dimethylformaide or N-Methylpyrrolidone. Typically, oligo- and polyimides are prepared at a dry weight of the monomers corresponding to about 10 to 40 wt%.

In the preparation of oligo- and polyimides, the monomers are mixed at ambient or at slightly elevated temperature, typically from about 25°C to 50°C, to obtain an oligo- or a polyamic acid as intermediate. Then, the oligo- or polyamic acid intermediate is imidized at a much higher temperature, such as about 180°C, by dehydration eliminating water.

Analogously to PEPA and EPA, compounds according to formula (I) or (II), such as PETA, may be, as readily understood by the skilled artesian, incorporated in different ways into oligo- and polyimides.

As an example, compounds according to formula (I) or (II) may be copolymerized into the polyimide by addition initially or at an early stage to a reaction mixture comprising di-amine and di-anhydride monomers to be polymerized. Examples of aromatic di-amines and di-anhydrides are given above.

As the formation of oligo- and polyimides involves formation of oligo- or polyamic acid intermediates, oligo- or polyamic acid intermediates end-capped with a compound according to formula (I) or (II) as well as oligo- or polyimide end-capped with a compound according to formula (I) or (II), may be isolated.

Compounds of formula (I) or (II) may also be reacted with an amino terminated oligo- or polyamic acid or an amino terminated oligo- or polyimide, respectively, after their preparation.

The reactivity of compounds according to formula (I) or (II), which are suitable for end-capping of amine terminated oligo- and polyimides, may be altered by reaction with compounds, such as aminofenols or aminoresorcinols, *O*-acetylated aminofenols or O-acetylated aminoresorcinols, aminobenzoic or aminophthalic acids or esters. Thus, compounds according to formula (I) or (II) may also be used to obtain cross-linkers for polyetherketones, polycarbonates etc.

As elaborated below, PETA may be synthesized from trimelletic anhydride and ethynylbensen, which both are readily available. Trimelletic anhydride may be obtained from pseudocumene (1,2,4-trimethylbenzene), while ethynylbenzene may be obtained from styrene or bromobenzene. Thus, PETA may be produced in large quantities, which is prerequisite to find use a cross-linker within the polymer industry. Further, the availability of suitable starting materials also means that price may be kept at a moderate level.

A further embodiment relates to a method of producing a monomer according to formula (II) as disclosed herein. Such a method comprises the step of:
- reacting trimelletic anhydride or a derivative thereof, such as trimellitic anhydride chloride, with a compound according to formula (IV) to obtain a monomer according to formula (I). 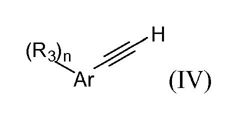
wherein "Ar", R3 and "n" have the meaning indicated above for formula (I) and (II).

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

Although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### Experimental

### Brief description of the drawings

Fig. 1 depicts a DSC-thermogram for EPA.
Fig. 2 depicts a DSC-thermogram for PEPA.
Fig. 3 depicts a DSC-thermogram for PETA.
Fig. 4 depicts a DSC-thermogram for a polyimid end-capped with PETA.

The following examples are mere examples and should by no mean be interpreted to limit the scope of the invention. Rather, the invention is limited only by the accompanying claims.

### Abbreviations

- PETA: Phenylethynyl trimelletic anhydride (5-(3-phenylprop-2-ynoyl)isobenzofuran-1,3-dione)
- PEPA: Phenylethynyl phtalic anhydride
- EPA: Ethynyl phtalic anhydride
- PETI: Phenylethynyl terminated imide oligomer
- BPDA: 3,3',4,4'-Biphenyl tetracarboxylic dianhydride
- 4,4-ODA: 4,4'-oxydianiline
- NMP: N-Methyl-2-pyrrolidone

### 5-(3-phenylprop-2-ynoyl)isobenzofuran-1,3-dione (PhenylEthynylTrimelleticAnhydride; PETA)

0.16 g of palladium acetate (0.78 mmol) was added to a mixture of 300 ml toluene, 54.6 ml triethyl amine (0.39 mol), 43 ml phenyl acetylene (0.39 mol) and 75 g oftrimellitic anhydride chloride (0.36 mol). The mixture was stirred at rt for 1.5 h, whereupon 16.5 ml triethyl amine (0.12 mol) was added. The mixture was stirred for additionally 1 h, whereupon the resulting solid was filtered of. The solid was washed with 150 ml toluene, and then suspended in 750 ml ethyl acetate. The mixture was refluxed for 30 min, whereupon it was filtered on a bed of silica gel. The filtrate was concentrated under vacuum, and the precipitate formed was filtered of, and washed with toluene. The solid was dried under vacuum to give 39 g of PETA (0.14 mol), ¹H NMR (400 MHz, CDCl₃): δ 8.84 (q, 1H, *J*= 1.4 Hz), 8.74 (dd, 1H, *J*= 7.8 Hz), 8.22 (dd, 1H, *J*= 8.0 Hz), 7.77 (m, 1H) 7.59 (m, 2H), 7.51 (m, 2H).

Prior to analysis, the product was hydrolysis in methanol containing 2 % sulfuric acid to provide two regio isomers. The regio isomers was analyzed by LC/LTV/MS performed on an Agilent 1100 system comprised of a degasser, binary pump, autosampler, single wavelength UV detector, operating at 254 nm, and a single quadrupole mass detector, equipped with an electrospray ionization source, operating in single ion monitoring. The mobile phase was H₂O:Methanol (50/50) with 0.1 % acetic acid and the separation column (150 mm * 2 mm) was packed with octadecyl coated silica particles with an average diameter of 3 µm. The two regio isomers, obtained by the hydrolysis of PETA, had retention factors of 2.64 and 2.93, respectively. Both isomers were found to form ions with m/z's: 277, 309 and 331.

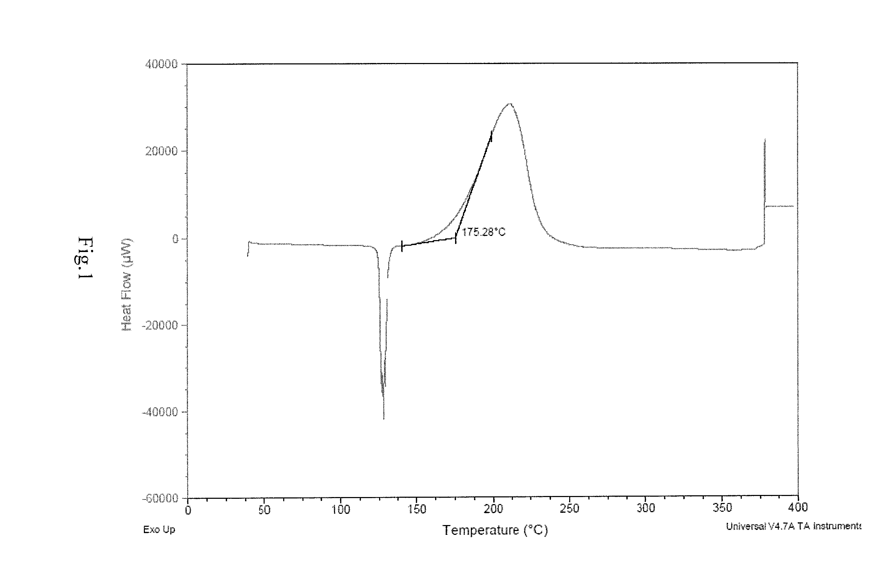

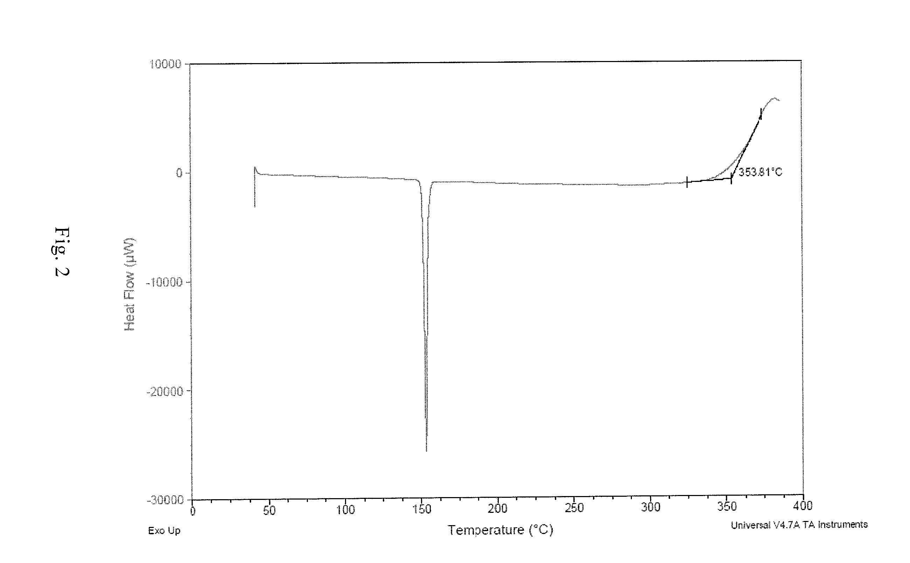

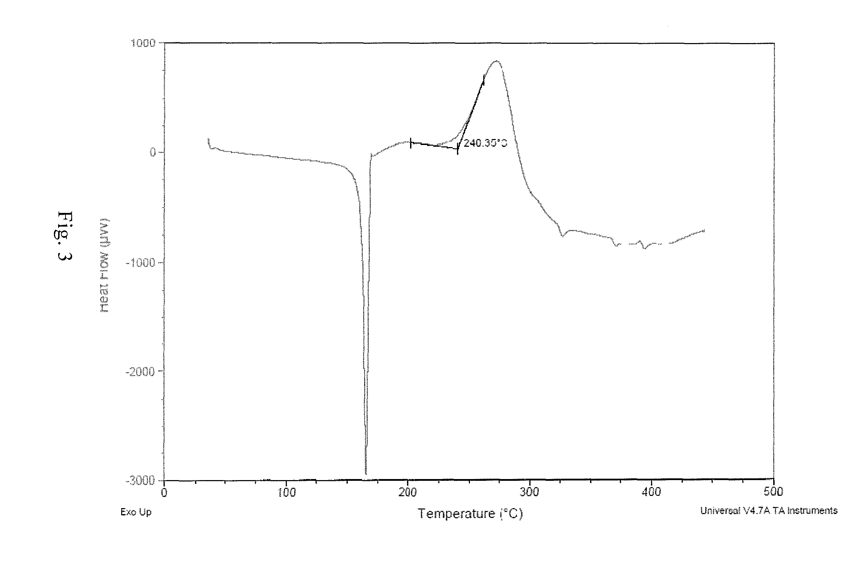

PETA, as well as PEPA and EPA, were analyzed by differential scanning calorimetry (DSC) using a TA instrument DSC Q2000 with a ramp of 50°C - 400°C (10°/min). As seen in Fig. 1-3, onset for curing was found to be about 175°C for EPA, about 240°C for PETA and about 355°C for PEPA. This confirms that PETA may be cross-linked at much lower temperatures compared to PEPA. Further, it is noted that while the curing temperature for PEPA and PETI are comparable, this is not the case for EPA.

### Polyimid end-capped with PETA

BPDA (25.0 g, 0.085 mmol), 4,4-ODA (15.3 g, 0.077 mmol), PETA (2.35, 0.0085 mmol) and acetic acid were mixed at room temperature over nitrogen atmosphere and heated to 100°C for two hours. NMP (350 mL) was added to the reaction mixture and the temperature was raised to 120°C for one hour during which the color of the reaction mixture turned orange. The temperature was further raised to 145°C, and acetic acid started to distill off from the reactor during 1.5 hours while the temperature was increased to 175°C. The reaction mixture was cooled down to room temperature and water (300 mL) was added. The mixture was filtered, washed with water (600 mL), washed with methanol (300 mL) and dried at 100°C over vacuum over night to give 33.5 g of a polyimid end-capped with PETA as an yellow solid (79 wt % recovery).

The obtained end-capped polymimid was analyzed by differential scanning calorimetry (DSC) using a TA instrument DSC Q2000. The heating profile employed was: Heat: 35°C => 390°C (10°/min); Cool: 390°C => 70°C (5°C/min); and Heat: 70°C =>400°C (10°/min).

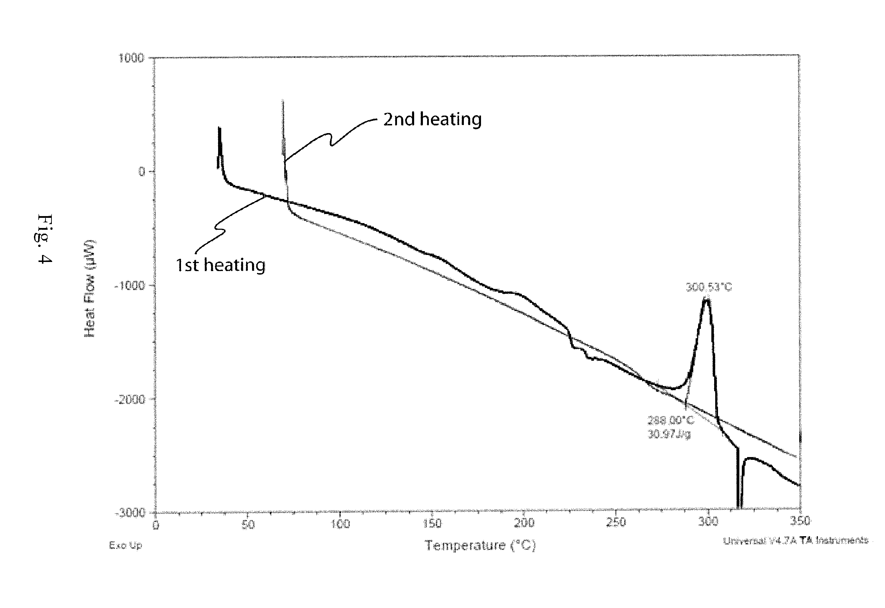

The DSC-thermogram (Fig. 4) of the polyimide end-capped with PETA clearly shows a curing exotherm at 270-300°C in the 1st heating which is not seen in the second heating, indicating complete cure of the product.

## Claims

1. A compound according to formula (I) or (II), 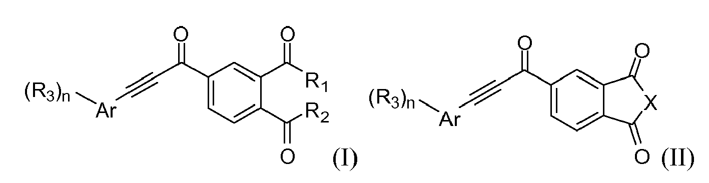 wherein
"Ar" is an aryl or a heteroaryl;
R1 and R2 are independently selected from the group consisting of OH, halo, OC1-C8 alkyl, NH2, NHC1-8 alkyl, N(C1-8 alkyl)₂, wherein said alkyl may be the same or different, OC(O)C1-8 alkyl, OCO-1 alkylenephenyl, and NHCO-1 alkylenephenyl;
R3 is, independently if "n" is 2 or more, selected from the group consisting of C1-4 alkyl, OC1-4 alkyl, halogen, cyano, nitro, C1-4 fluoroalkyl;
the substituent(s) R3 may be connected to any substitutable atom(s) of "Ar"; "n" is an integer from 0 (zero) to 5; and
"X" is selected from the group consisting of O (oxygen), NH, N-phenyl, N-benzyl, and N-C1-8 alkyl.

2. The compound according to claim 1, wherein "Ar" is phenyl, naphtyl, thiophenyl or furanyl.

3. The compound according to claim 1, wherein "Ar" is phenyl.

4. The compound according to any one of the claims 1 to 3, wherein the integer "n" is 0.

5. The compound according to any one of the claims 1 to 4, wherein n" is 1 or more, and R3 is, independently if "n" is 2 or more, selected from the group consisting of methyl, methoxy, nitro, and trifluoromethyl.

6. The compound according to any one of the claims 1 to 5, wherein said compound is a compound according to formula (II) and "X" is O (oxygen).

7. The compound according to claim 6, wherein said compound is

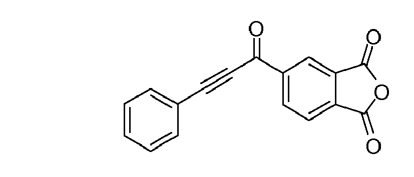

8. The compound according to any one of the claims 1 to 5, wherein said compound is a compound according to formula (I) and R1 and R2 are selected from OH, chloro, and OC1-C8 alkyl.

9. An oligo- or polyimide comprising a residue according to formula (III), 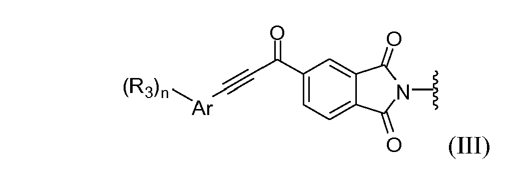 wherein the waved line indicates the point of attachment to the oligo- or polyimide, and "Ar", "n", and R3 have the same meaning as in any one of the claims 1 to 5.

10. The oligo- or polyimide according to claim 9, wherein said oligo- or polyimide comprises at least one residue of a monomer selected from the group consisting of pyromellitic dianhydride, 4,4'-oxydiphthalic anhydride, 2,2-bis-[4-(3,4-dicarboxyphenoxy)phenyl]-propane dianhydride, 3,3',4,4'-benzophenonetetracarboxylic acid dianhydride and 3,3',4,4'-tetracarboxybiphenyl dianhydride and at least one residue of a monomer selected from the group consisting of 4,4'-oxydianiline, 1,4-diaminobenzene, 1,3-diaminobenzene, 1,3-bis-(4-aminophenoxy)benzene, 1,3-bis-(3-aminophenoxy)benzene, methylenedianiline, and 3,4'-oxydianiline.

11. The oligo- or polyimide according to claim 9 or 10, having a number average molecular weight of about 1,000 to 20,000.

12. A composition comprising an oligo- or polyimide according to any one of the claims 9 to 11.

13. The composition according to claim 12, further comprising an additional polymer, and/or at least one filler, reinforcement, pigment, and/or plasticizer.

14. The composition according to claim 12 or 13, wherein the oligo- or polyimide is present in an amount corresponding to at least 10 wt%.

15. A method of producing a compound according to formula (II) according to any of one of the claims 1 to 6, the method comprising the step of:
- reacting trimellitic anhydride chloride with a compound according to formula (IV) 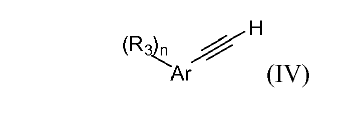 wherein "Ar", the integer "n" and R3 have the same meaning as in any one of the claims 1 to 5.
